# EUROPEAN PATENT SPECIFICATION

(11) **EP 4 233 867 B1**
(45) Date of publication and mention of the grant of the patent: **03.09.2025**
(21) Application number: 22158443.6
(22) Date of filing: 24.02.2022
(51) Int. Cl.: A61K 31/506, A61K 31/573, A61K 45/06, A61K 9/00, A61P 27/02

(54) **PDE5 INHIBITOR FOR USE IN THE TREATMENT OF ANTERIOR ISCHEMIC OPTIC NEUROPATHY**
PDE5 HEMMER ZUR VERWENDUNG BEI DER BEHANDLUNG DER ANTERIOREN ISCHÄMISCHEN OPTISCHEN NEUROPATHIE
INHIBITEUR DE PDE5 POUR UTILISATION DANS LE TRAITEMENT DE LA NEUROPATHIE OPTIQUE ISCHÉMIQUE ANTÉRIEURE

(43) Date of publication of application: 30.08.2023
(73) Proprietor: Nicox SA, 06410 Biot (FR)
(72) Inventor: IMPAGNATIELLO, Francesco, 20131 Milano (IT); BASTIA, Elena, 20121 Milano (IT); RONSIN, Gael, 67000 Strasbourg (FR)
(74) Representative: Bianchetti & Minoja with Trevisan & Cuonzo IPS SRL

(56) References cited:
- WO-A1-2020/030489
- WO-A2-01/10406
- US-A1- 2006 014 754
- BASTIA ELENA ET AL: "NCX 1741, a Novel Nitric Oxide-Donating Phosphodiesterase-5 Inhibitor, Exerts Rapid and Long-Lasting Intraocular Pressure-Lowering in Cynomolgus Monkeys", vol. 37, no. 4, 1 May 2021 (2021-05-01), US, pages 215 - 222, XP055803290, ISSN: 1080-7683, Retrieved from the Internet <URL:https://www.liebertpub.com/doi/pdf/10.1089/jop.2020.0126> DOI: 10.1089/jop.2020.0126
- VARMA R S ET AL: "EFFECTS OF NITRIC OXIDE DONORS ON THE RETINAL FUNCTION MEASURED WITH ELECTRORETINOGRAPHY", JOURNAL OF OCULAR PHARMACOLOGY AND THERAPEUTICS, MARY ANN LIEBERT, INC., NEW YORK, NY, US, vol. 16, no. 6, 1 January 2000 (2000-01-01), pages 571 - 578, XP009021772, ISSN: 1080-7683
- BASTIA ELENA ET AL: "NCX 470 Restores Ocular Hemodynamics and Retinal Cell Physiology After ET-1-Induced Ischemia/Reperfusion Injury of Optic Nerve and Retina in Rabbits", JOURNAL OF OCULAR PHARMACOLOGY AND THERAPEUTICS, 4 July 2022 (2022-07-04), US, XP055942368, ISSN: 1080-7683, DOI: 10.1089/jop.2022.0004

## Description

The present invention concerns the topical ophthalmic use of [(2S)-1-(4-{[(3-chloro-4-methoxyphenyl)ethyl]amino}-5-{[(pyrimidin-2-yl)methyl]carbamoyl}pyrimidin-2-yl)pyrrolidin-2-yl]methyl 6-(nitrooxy)hexanoate for the treatment of anterior ischemic optic neuropathy as it improves the perfusion to the optic nerve whereby leading to an improvement in visual function.

Anterior ischemic optic neuropathy (AION) is characterized by dysfunctional optic nerve and retina, it is mostly a pathology affecting elderly population, often resulting in a significant loss of visual acuity. There are 2 types of AION: arteritic and non-arteritic. The Arteritic form (AAION) is associated with giant cell arteritis which is described as an inflammation of large vessels that affects the lining of the arteries and leads to vessel swelling and reduction of blood flow that results in ischemia of the optic nerve and retina.

The Non-arteritic form (NAION) is more frequent and the reduced blood flow causing the damage to the optic nerve and retina is not associated with local pathological processes of the blood vessels (e.g. inflammation, clot) but it is rather dependent on cardiovascular risk factors including diabetes, vasospasm and impaired autoregulation, nocturnal hypotension, and sleep apnea.

The visual loss in both forms of AION is usually permanent, with partial spontaneous recovery possibly occurring within the initial weeks or months from its diagnosis.

Nowadays there is no unique and well-established treatment strategy for AION. Oral corticosteroids are sometime used for AION. However, the systemic side effects of corticosteroids largely hamper their effectiveness and limit their use. Off-label intravitreal corticosteroids have also been proposed and sometime used. Dexamethasone as well as other corticosteroids administered *via* intravitreal injection/implant, although have less systemic side effects, they all result in a robust rise in intraocular pressure. Furthermore, cataract formation after corticosteroids ocular dosing has been reported. Last, corticosteroids treatment has been reported to evoke vessel constriction and vasospasm thereby potentially worsening the ischemic insult observed in AION patients. Anti-VEGF therapy has been shown to inhibit inflammation and reduce edema in the eye; however, these compounds have the potential to exacerbate vascular dysfunction in the eye and, consequently, further reduce ocular perfusion.

WO 01/10406 describes the treatment of ocular disorders by agents that enhance ocular blood flow to and around the optic nerve and that are selected from compounds that activate guanylate cyclase or stimulate nitric oxide synthase, and nitric oxide donors, nitrosated and /or nitrosylated PDE inhibitors, PDE type 5 and type 6 inhibitors. WO 01/10406 specifically mentions the nitrosated and/or nitrosylated PDE inhibitors disclosed in US 5,958,926 and US 5,874,437 which are specifically focused on the use of nitrosated and/or nitrosylated PDE inhibitors for treating sexual dysfunction. Neither WO 01/10406, nor the above two US patents provide any experimental results regarding the efficacy of the cited nitrosated and/or nitrosylated PDE inhibitors in increasing ocular blood flow. The experimental results described in WO 01/10406 refer to clinical studies conducted on patients treated with orally administered Sildenafil citrate, Sildenafil citrate was administered topically to the eye only in the study conducted on a subject with normal visual function, therefore WO 01/10406 does not report any information related to the efficacy of the compounds that act by increasing NO levels and/or inhibiting phosphodiesterase 5 in a pathological condition in which the retinal vessel and tissue are impaired.

US 6,462,066 discloses a method for the treatment or prophylaxis of neuropathies including, inter alia, ischemic optic neuropathy by administering a compound that decreases the cytosolic Ca²⁺ concentration and inhibits the intracellular calcium-mediated neuronal damage caused by reperfusion injury. This compound is selected from dantrolene, aminodantroline, azumolene, cyclopiazonic acid or 2,5-di(tertbutyl)-1,4-benzohydroquinone.

US 10,159,669 discloses topical or intravitreal ophthalmic use of Mdivi-1 and Nutlin-3, individually or in combination for the treatment of ischemic optic neuropathies.

This reference discloses that Nutlin-3 inhibits apoptosis by inhibiting Bax and Bak in the apoptosis pathway and Mdivi-1 regulates apoptosis via regulating mitochondrial fission or fusion and the combination of these drugs blocks Bax/Bak and Drpl interaction on the mitochondria surface as a key step in the apoptotic pathway of various ophthalmic diseases including glaucoma, ischemic optic neuropathy, hereditary optic neuropathy and retinal artery/vein occlusions.

EP 0 968 716 discloses that Nicardipine, which is a systemic calcium antagonist, has a blood flow increasing action in retinochoroid and inhibits vasocontraction caused by ET-1, which is one of the biological vasocontracting substances in the eye, without increasing intraocular pressure.

This reference suggests that nicardipine may be suitable for the treatment of ocular diseases associated with insufficient retinal blood flow including ischemic optic neuropathies.

Patent applications US 2002/0119974 and US 2006/0014754 disclose that oral administration of a cyclic guanosine 3',5'-monophosphate phosphodiesterase type 5 (PDE5 inhibitor) of the class of pyrazolo[4,3-d]pyrimidin-7-one, in particular Sildenafil, may be used to prevent or treat diseases or disorders of the eye including optic neuropathy such as ischemic optic neuropathy and glaucomatous optic neuropathy. These references disclose that the administration of a PDE5 inhibitor increases the level of cGMP which, in turn, increases blood flow to the optic nerve and retina. However, these references fail to provide any experimental results.

Patent application US 2002/0168424 discloses the use of a topical ophthalmic mixture of a nitric oxide (NO) donor and the specific phosphodiesterase type 5 (PDE5 inhibitor) sildenafil citrate for treating glaucoma or ocular hypertension, according to this reference the nitric oxide (NO) donor and the phosphodiesterase type 5 (PDE5 inhibitor) works synergistically to lower intraocular pressure. The authors also mentioned that as a consequence of relaxation of trabecular meshwork (TM) and Schlemm's canal (SC) leading to enhanced aqueous humor (AH) drainage, there would be an increase in blood circulation to the optic nerve. However, how the two effects relate to each other is not discussed or shown experimentally. This aspect is particularly important as the two effects occur via two independent target tissues, TM/SC versus blood vessels.

Neurobiology of Disease 121 (2019) 65-75 discloses that Tadalafil, a PDE5 inhibitor, prevented IOP-induced degeneration of retinal ganglion cells (RGCs) in two murine models of, respectively, primary open angle glaucoma (POAG) and primary angle closure glaucoma (PACG). The result of in vitro studies showed that the RGCs neuroprotective effect was IOP-independent and it was promoted by the increase of cGMP bioavailability that, in turns, improved survival of RGCs through modulation of pro- and anti-apoptotic pathways. This reference seems to suggest that the RGCs neuroprotection may be mediated by an additional effect of tadalafil on retinal vascular function, nevertheless no experimental data showing the possible effect of tadalafil on retinal vasculature are reported.

WO 2021/245192 discloses NO-releasing PDE5 inhibitors and their potential therapeutic uses for treating, among others, ophthalmic diseases like glaucoma, diabetic retinopathy, macular degeneration including age dependent macular degeneration and degenerative eye diseases associated with microvascular disorders. The reported experimental data show that the NO-releasing PDE5 inhibitors enhanced the accumulation of cGMP presumably by activating soluble guanylate cyclase (cGC) and inhibiting its degradation *via* PDE5 inhibition. While the data and the cell line used (trabecular meshwork cells) are consistent with an activity of this class of compounds on aqueous humor drainage and intraocular pressure reduction, the effectiveness of these compounds on other targets in different tissue compartments such as vessels, responsible of ocular blood flow modulation, is not disclosed.

British J Ophthalmology 83 (1999) 162-167, discusses the effect of the NO donor isosorbide mononitrate (ISMN) on blood flow nearby the optic nerve head and choroid after systemic dosing. Authors conclude that under their experimental conditions ISMN increases blood flow nearby optic nerve head without changes to the choroidal compartment. The results of this work do not suggest that the ocular effect of ISMN systemic dosing may be reproduced by ocular topical application of ISMN; the availability of ISMN and its conversion to active nitric oxide may not be equal to that observed after systemic treatment.

[(2S)-1-(4-{[(3-Chloro-4-methoxyphenyl)methyl]amino}-5-{[(pyrimidin-2-yl) methyl]carbamoyl}pyrimidin-2-yl)pyrrolidin-2-yl]methyl 6-(nitrooxy)hexanoate of formula (1) is a nitric oxide donating derivative of the phosphodiesterase type 5 inhibitor, avanafil. 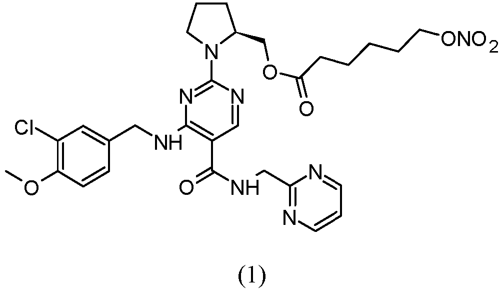 [(2S)-1-(4-{ [(3 -Chloro-4-methoxyphenyl)methyl]amino}-5-{ [(pyrimidin-2-yl)methyl] carbamoyl}pyrimidin-2-yl)pyrrolidin-2-yl]methy]6-(nitrooxy)hexanoate is also referred to herein as Compound (I).

Compound (I) is disclosed in WO 2020/030489 that relates to NO-donating PDE5 inhibitors and their effect in reducing intraocular pressure. This reference discloses the use of the NO-donating PDE5 inhibitors for the treatment of ocular diseases associated with elevated intraocular pressure, such as ocular hypertension and glaucoma, and for treating retinopathies such as retinopathy of prematurity, retinal vein occlusion and diabetic macular edema. WO 2020/030489 does not disclose or suggest the effect of the NO-donating PDE5 inhibitors on ischemic insults of the optic nerve head and retina or, specifically, on a disease such as AION.

Journal of Ocular Pharmacology and Therapeutics, Vol. 37, 4, 215-2022, 2021, discloses that topically administered NCX 1741, which is the citrate salt of Compound (I), lowers IOP in a model of intraocular pressure increase in non-human primates. This effect results from the concomitant activity of NO that elicit cGMP formation and, Avanafil that inhibits its breakdown in target tissues.

WO 2021/156275 discloses the use of [(2S)-1-(4-{[(3-chloro-4-methoxyphenyl) methyl]amino}-5-{[(pyrimidin-2-yl)methyl]carbamoyl}pyrimidin-2-yl)pyrrolidin-2-yl]methyl 6-(nitrooxy)hexanoate in combination with a prostaglandin analog to decrease elevated intraocular pressure. The therapeutic use of this combination is based on the complementary mode of action of the two compounds namely: the prostaglandin analog reduces IOP via uveoscleral aqueous humor drainage and NO-donating PDE5 inhibitor induces drainage of the aqueous humor through the trabecular meshwork upon the release of nitric oxide. Also this reference fails to disclose or suggest the effect of any NO-donating PDE5 inhibitor on markers related to the ischemic insults of the optic nerve head and retina or, specifically, on a disease such as AION.

Currently, there is still no approved therapy to specifically treat anterior ischemic optic neuropathy; only off-label treatments are used.

The references to methods of treatment in this description are to be interpreted as references to the compound, pharmaceutical compositions and medicaments of the present invention for use in a method for treatment of the human body by therapy.

The inventors found that Compound (I) reduced ophthalmic artery resistive index (OA-RI) and increased photoreceptor activity in a model of ischemia/reperfusion injury of the optic nerve induced by the subtenon injection of Endothelin-1 (ET-1) in rabbits. The Resistive Index (RI) is a widely used measure of resistance to arterial flow; elevation of the RI of ophthalmic artery indicates an increase of peripheral resistance or a vasospasm that may result in decrease in blood flow to optic nerve and retina. ET-1 is a potent vasoconstrictor released from endothelial cells. Elevated levels of ET-1 are associated with ocular circulation disorder, decrease of optic disc blood flow and deterioration of visual function. The experimental data indicate that Compound (I) is able to ameliorate ocular perfusion and retinal function, therefore Compound (I) may be an effective therapeutic approach to treat anterior ischemic optic neuropathy that is a disorder associated with hypoperfusion or lack of perfusion of optic nerve head.

The present invention relates to a method for treating anterior ischemic optic neuropathy comprising administering [(2S)-1-(4-{[(3-chloro-4-methoxyphenyl)methyl] amino}-5-{[(pyrimidin-2-yl)methyl]carbamoyl}pyrimidin-2-yl)pyrrolidin-2-yl]methyl 6-(nitrooxy)hexanoate (Compound (I)), as defined in the claims.

Object of the invention is the subject-matter defined in claims 1 to 6.

An embodiment of the present invention provides [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy)hexanoate for use in the treatment of anterior ischemic optic neuropathy in a patient in need thereof wherein [(2S)-1-(4-{[(3-chloro-4-methoxyphenyl)methyl]amino}-5-{ [(pyrimidin-2-yl)methyl]carbamoyl}pyrimidin-2-yl)pyrrolidin-2-yl]methyl 6-(nitrooxy)hexanoate is topically applied to the eye or it is administered to the eye by subconjunctival injection.

Another embodiment of the present invention provides [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy)hexanoate for use in the treatment of non-arteritic anterior ischemic optic neuropathy (NAION) in a patient in need thereof wherein [(2S)-1-(4-{[(3-chloro-4-methoxyphenyl)methyl]amino}-5-{[(pyrimidin-2-yl)methyl]carbamoyl} pyrimidin-2-yl)pyrrolidin-2-yl]methyl 6-(nitrooxy)hexanoate is topically applied to the eye or it is administered to the eye by subconjunctival injection.

Another embodiment of the present invention provides [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy)hexanoate for use in the treatment of arteritic anterior ischemic optic neuropathy (AAION) in a patient in need thereof wherein [(2S)-1-(4-{[(3-chloro-4-methoxyphenyl)methyl]amino}-5-{[(pyrimidin-2-yl)methyl]carbamoyl} pyrimidin-2-yl)pyrrolidin-2-yl]methyl 6-(nitrooxy)hexanoate is topically applied to the eye or it is administered to the eye by subconjunctival injection.

Another embodiment of the present invention provides [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy)hexanoate for use in the treatment of anterior ischemic optic neuropathy in a patient in need thereof wherein [(2S)-1-(4-{[(3-chloro-4-methoxyphenyl)methyl]amino}-5-{ [(pyrimidin-2-yl)methyl]carbamoyl}pyrimidin-2-yl)pyrrolidin-2-yl]methyl 6-(nitrooxy)hexanoate is topically applied to the eye or it is administered to the eye by subconjunctival injection and the patient is receiving a concomitant treatment with corticosteroids.

The method of treating of anterior ischemic optic neuropathy of the present invention has multiple advantages: i) it is safer than oral, intravenous or intravitreal treatment with corticosteroids or anti-VEGF because it reduces the liability to encounter systemic side-effects or serious adverse events due to repeated intravitreal injections such as endophthalmitis, retinal detachments, ocular inflammation and hemorragies; ii) it is non-invasive and does not require any additional chirurgic practice because Compound (I) can be self-dosed by patients as topical eye drops (collirium). iii) this treatment strategy is expected to enhance patients' compliance.

In addition, the intraocular pressure lowering ability of Compound (I) may progressively reverse the changes in intraocular pressure consequent to ischemia/reperfusion injury, thus further reducing the risk of progression of AION, as well as counteract the intraocular pressure elevation induced by corticosteroids currently used off-label in the AION therapy.

Treatment of anterior ischemic optic neuropathy includes the treatment of patients holding signs or symptoms of acute arteritic and non-arteritic ischemic optic neuropathy and the treatment of patients after the disease has already been diagnosed so to maintain or ameliorate visual acuity or visual field and preventing additional loss of vision.

[(2S)-1-(4-{[(3-Chloro-4-methoxyphenyl)methyl]amino}-5-{[(pyrimidin-2-yl) methyl]carbamoyl}pyrimidin-2-yl)pyrrolidin-2-yl]methyl 6-(nitrooxy)hexanoate is generally administered for treating anterior ischemic optic neuropathy to a person in need thereof in an amount ranging from 5µg/eye (0.01%, 50microL/eye) to 5000µg/eye (10%, 50microL/eye), preferably in an amount ranging from 50µg/eye (0.1%, 50microL/eye) to 1000µg/eye (2%, 50microL/eye) and, most preferably in an amount of 250µg/eye (0.5%, 50microL/eye) and 1000 µg/eye (2%, 50microL/eye).

Compound (I) is administered as pharmaceutical compositions adapted for topical administration to the eye or in the form of subconjunctival ocular injections in which the Compound (I) is combined with a suitable ophthalmic vehicle.

Pharmaceutical compositions adapted to ocular topical administration include eye drops (solutions or suspensions) or eye ointments.

Thus the present invention also provides an ophthalmic composition comprising Compound (I) and a pharmaceutically acceptable vehicle and/or carrier for use in the treatment of anterior ischemic optic atrophy, as defined in the claims.

The topically ophthalmic dosage forms can be produced by admixing pharmaceutical acceptable excipients and vehicles typically necessary for usual preparation, and processing according to conventional methods. For example, pharmaceutical acceptable excipients to be used for an eye drop include the following: buffering agents, isotonizing agents, preservatives, surfactants, water soluble polymers. The pH of an eye drop is generally set to about 3 to 7, preferably 4 to 6.

### EXAMPLE 1

These experiments were performed to determine the effects of [(2S)-1-(4-{[(3-chloro-4-methoxyphenyl)methyl]amino}-5-{[(pyrimidin-2yl)methyl]carbamoyl} pyrimidin-2-yl)pyrrolidin-2-yl]methyl 6-(nitrooxy)hexanoate (Compound (I)) on ocular vascular reactivity and retinal function after ocular topical dosing in a model of ischemia/reperfusion injury of the optic nerve induced by the subtenon injection of endothelin-1 (ET-1) in rabbits.

[(2S)-1-(4-{[(3-chloro-4-methoxyphenyl)methyl]amino}-5-{[(pyrimidin-2yl)methyl] carbamoyl}pyrimidin-2-yl)pyrrolidin-2-yl]methyl 6-(nitrooxy)hexanoate can be synthetized as disclosed in example 1 of WO 2020/030489.

### Method and treatment

Adult male New Zealand white (NZW) rabbits weighting 1.5-2.0 kg were used. All animals received a single 30microL/eye topical dose of Compound (I) (1% w/w) or vehicle (Kolliphor^{®} EL 5.0% w/w, Myrj^{™} S40 3.0% w/w, Kollisolv^{®} PEG400 2.8% w/w, H₃BO₃ 0.19% w/w, Na₂HPO₄·7H₂O 0.51% w/w, EDTA 0.10% w/w, BAK 0.01% w/w, HCl 0.5N q.s. pH 6.6, purified water q.s. 100g) twice daily for 4 consecutive weeks starting 2 weeks after Endothelin-1 (ET-1) first dosing.

Ischemia/reperfusion injury was induced in each animal by subtenon injection (twice/week for 6 weeks) of 200 microL of 250 nM ET-1 dissolved in water using a lacrimal cannula under anaesthesia produced by ketamine and xylazine injected intramuscularly.

Ophthalmic Artery Resistive Index (OA-RI) measurements were taken using an Echo Color Doppler prior to ET-1 treatment (baseline, time 0), and weekly thereafter till the end of the study. Pourcelot resistive index for ophthalmic artery (OA-RI) was calculated using the following formula: (OA-PSV - OA-EDV) / OA-PSV where OA-PSV and OA-EDV refer to Ophthalmic Artery Peak Systolic Velocity and Ophthalmic Artery End Diastolic Velocity, respectively.

Electroretinogram (ERGs) recording took place under topical anaesthesia. The eyes were also dilated by topical application of tropicamide 1% and, when needed, adapted to darkness for at least 2 hours prior to ERGs recording. ERGs recording were performed with standard contact lens corneal electrodes. Specifically, the dark-adapted 0.01 ERG (mostly rod response), dark-adapted 3.0 ERG (combined rod/cone response) and, light-adapted 3.0 (mostly cone response) were recorded.

Measurements were taken prior to ET-1 first dose (baseline, time 0), then at the end of week 2 (prior to vehicle- or Compound (I) first day-first dose) and at the end of week 6.

### Results

### Ophthalmic Artery Resistive Index (OA-RI)

The Resistive Index (RI) is a measure of resistance to arterial flow, elevation of the RI indicates an increase of peripheral resistance or a vasospasm.

Prior to endothelin-1 (ET-1) dosing OA-RI were 0.35±0.09 and 0.40±0.08 respectively in animals randomized to vehicle or Compound (I) treatments (Table 1). Twice weekly dosing with ET-1 for 2 weeks elevated OA-RI. In the animals treated with vehicle, OA-RI continued increasing over the following 4 weeks. In animals treated with Compound (I) OA-RI showed a decrease (0.43±0.09 and 0.43±0.04 on week 4 and 6, respectively) (Table 1) likely as a result of the compensatory effect of Compound (I) on the effects of ET-1. Data are shown as mean±SD.

| **Table 1: Ophthalmic artery resistive index (OA-RI) values at different time-points** | | | | |
|---|---|---|---|---|
| **Test items** | **Baseline** | **Dosing schedule** | | |
| | | **ET-1 (2 weeks)** | **ET-1 (4-weeks) + Test items (2 weeks daily bid)** | **ET-1 (6 weeks) + Test items (4 weeks daily bid)** |
| Cpd (I) | 0.40±0.08 | 0.48±0.06 | 0.43±0.09 (p=0.05) | 0.43±0.04 (p<0.05) |
| Vehicle | 0.35±0.09 | 0.51±0.07 | 0.52±0.05 | 0.54±0.07 |
| Cpd (I) = Compound (I) | | | | |

### Electroretinogram (ERG)

ERG is a test that provides a quantitative measurement of central retinal function.

ET-1 treatment resulted in a decline in retinal functions two weeks after the injection of ET-1. However, eyes treated for 4 weeks with Compound (I) exhibited less impairment in the ERG wave amplitude than those treated with vehicle.

| **Table 2: Electroretinogram (ERG) response at different time-points** | | | | |
|---|---|---|---|---|
| **Test items** | **ERG stimuli** | **ERG Amplitude (µV ± S.D.)** | | |
| | | **Baseline** | **ET-1 (2 weeks)** | **ET-1 (6 weeks) + Test items (4 weeks daily bid)** |
| Vehicle | Light adapted 3.0 (Rod/cone response) | 115.6±46.2 | 100.7±31.8 | 107.2±19.0 |
| Cpd (I) | | 115.9±30.0 | 100.5± 33.3 | 131.6±46.4 |
| Cpd (I) = Compound (I) | | | | |

In summary, the results showed that Compound (I) ameliorates ocular vascular reactivity i.e. ophthalmic artery resistive index (OA-RI) and retinal function i.e. ERG after repeated ocular topical dosing in a well-defined model of ischemia/reperfusion injury of the optic nerve induced by the subtenon injection of ET-1 in rabbits.

These data provide evidence of an improved ocular perfusion and retinal cell physiology after Compound (I) dosing which may ultimately lead to reverse optic nerve degeneration and retina cell dysfunction consequent to repeated ischemic lesions.

## Claims

1. [(2S)-1-(4-{[(3-chloro-4-methoxyphenyl)methyl]amino}-5-{[(pyrimidin-2-yl) methyl]carbamoyl}pyrimidin-2-yl)pyrrolidin-2-yl]methyl 6-(nitrooxy)hexanoate for use in the treatment of anterior ischemic optic neuropathy in a patient in need thereof wherein [(2S)-1-(4-{[(3-chloro-4-methoxyphenyl)methyl]amino}-5-{[(pyrimidin-2-yl)methyl] carbamoyl }pyrimidin-2-yl)pyrrolidin-2-yl]methyl 6-(nitrooxy)hexanoate is administered to the eye topically or by subconjuntival injection.

2. [(2S)-1-(4-{[(3-chloro-4-methoxyphenyl)methyl]amino}-5-{[(pyrimidin-2-yl) methyl]carbamoyl}pyrimidin-2-yl)pyrrolidin-2-yl]methyl 6-(nitrooxy)hexanoate for use in the treatment according to claim 1 wherein the anterior ischemic optic neuropathy is non-arteritic anterior ischemic optic neuropathy.

3. [(2S)-1-(4-{[(3-chloro-4-methoxyphenyl)methyl]amino}-5-{[(pyrimidin-2-yl) methyl]carbamoyl}pyrimidin-2-yl)pyrrolidin-2-yl]methyl 6-(nitrooxy)hexanoate for use in the treatment according to claim 1 wherein anterior ischemic optic neuropathy is arteritic anterior ischemic optic neuropathy.

4. [(2S)-1-(4-{[(3-chloro-4-methoxyphenyl)methyl]amino}-5-{[(pyrimidin-2-yl) methyl]carbamoyl}pyrimidin-2-yl)pyrrolidin-2-yl]methyl 6-(nitrooxy)hexanoate for use in the treatment according to any of claims 1 to 3 wherein [(2S)-1-(4-{[(3-chloro-4-methoxyphenyl)methyl]amino}-5-{[(pyrimidin-2-yl)methyl]carbamoyl}pyrimidin-2-yl)pyrrolidin-2-yl]methyl 6-(nitrooxy)hexanoate is administered as ophthalmic pharmaceutical compositions.

5. [(2S)-1-(4-{[(3-chloro-4-methoxyphenyl)methyl]amino}-5-{[(pyrimidin-2-yl) methyl]carbamoyl}pyrimidin-2-yl)pyrrolidin-2-yl]methyl 6-(nitrooxy)hexanoate for use in the treatment according to any of claims 1 to 3 wherein the patient is receiving a concomitant treatment with corticosteroids.

6. An ophthalmic composition comprising [(2S)-1-(4-{[(3-chloro-4-methoxyphenyl)methyl]amino}-5-{[(pyrimidin-2-yl) methyl]carbamoyl}pyrimidin-2-yl)pyrrolidin-2-yl]methyl 6-(nitrooxy)hexanoate and a pharmaceutically acceptable vehicle and/or carrier for use in the treatment of anterior ischemic optic neuropathy in a patient in need thereof, wherein said composition is administered to the eye topically or by subconjuntival injection.

## Patentansprüche

1. [(2S)-1-(4-{[(3-Chlor-4-methoxyphenyl)methyl]amino}-5-{[(pyrimidin-2-yl)methyl]carbamoyl}pyrimidin-2-yl)pyrrolidin-2-yl]methyl-6-(nitrooxy)hexanoat zur Verwendung bei der Behandlung von anteriorer ischämischer optischer Neuropathie bei einem Patienten, der diese benötigt, wobei [(2S)-1-(4-{[(3-Chlor-4-methoxyphenyl)methyl]amino}-5-{[(pyrimidin-2-yl)methyl]carbamoyl}pyrimidin-2-yl)pyrrolidin-2-yl]methy1-6-(nitro-oxy)hexanoat dem Auge topisch oder durch subkonjuktive Injektion verabreicht wird.

2. [(2S)-1-(4-{[(3-Chlor-4-methoxyphenyl)methyl]amino}-5-{[(pyrimidin-2-yl)methyl]carbamoyl}pyrimidin-2-yl)pyrrolidin-2-yl]methyl-6-(nitrooxy)hexanoat zur Verwendung bei der Behandlung nach Anspruch 1, wobei die anteriore ischämische optische Neuropathie nicht-arteriotische anteriore ischämische optische Neuropathie ist.

3. [(2S)-1-(4-{[(3-Chlor-4-methoxyphenyl)methyl]amino}-5-{[(pyrimidin-2-yl)methyl]carbamoyl}pyrimidin-2-yl)pyrrolidin-2-yl]methyl-6-(nitrooxy)hexanoat zur Verwendung bei der Behandlung nach Anspruch 1, wobei die anteriore ischämische optische Neuropathie arteriotische anteriore ischämische optische Neuropathie ist.

4. [(2S)-1-(4-{[(3-Chlor-4-methoxyphenyl)methyl]amino}-5-{[(pyrimidin-2-yl)methyl]carbamoyl}pyrimidin-2-yl)pyrrolidin-2-yl]methyl-6-(nitrooxy)hexanoat zur Verwendung bei der Behandlung nach einem der Ansprüche 1 bis 3, wobei [(2S)-1-(4-{[(3-Chlor-4-methoxyphenyl)methyl]amino}-5-{[(pyrimidin-2-yl)methyl]carbamoyl}pyrimidin-2-yl)pyrrolidin-2-yl]methyl-6-(nitrooxy)hexanoat als ophthalmische pharmazeutische Zusammensetzungen verabreicht wird.

5. [(2S)-1-(4-{[(3-Chlor-4-methoxyphenyl)methyl]amino}-5-{[(pyrimidin-2-yl)methyl]carbamoyl}pyrimidin-2-yl)pyrrolidin-2-yl]methyl-6-(nitrooxy)hexanoat zur Verwendung bei der Behandlung nach einem der Ansprüche 1 bis 3, wobei der Patient eine begleitende Behandlung mit Corticosteroiden erhält.

6. Ophthalmische Zusammensetzung, umfassend [(2S)-1-(4-{[(3-Chlor-4-methoxyphenyl)methyl]amino}-5-{[(pyrimidin-2-yl)methyl]carbamoyl}pyrimidin-2-yl)pyrrolidin-2-yl]methyl-6-(nitrooxy)hexanoat und ein pharmazeutisch verträgliches Vehikel und/oder einen pharmazeutisch verträglichen Träger zur Verwendung bei der Behandlung von anteriorer ischämischer optischer Neuropathie bei einem Patienten, der diese benötigt, wobei die Zusammensetzung dem Auge topisch oder durch subkonjuktive Injektion verabreicht wird.

## Revendications

1. 6-(nitrooxy)hexanoate de [(2S)-1-(4-{[(3-chloro-4-méthoxyphényl)méthyl]amino}-5-{[(pyrimidin-2-yl)méthyl]carbamoyl}pyrimidin-2-yl)pyrrolidin-2-yl]méthyle pour une utilisation dans le traitement de la neuropathie optique ischémique antérieure chez un patient en ayant besoin, dans lequel le 6-(nitrooxy)hexanoate de [(2S)-1-(4-{[(3-chloro-4-méthoxyphényl)méthyl]amino}-5-{[(pyrimidin-2-yl)méthyl]carbamoyl}pyrimidin-2-yl)pyrrolidin-2-yl]méthyle est administré à l'œil par voie topique ou par injection sous-conjonctivale.

2. 6-(nitrooxy)hexanoate de [(2S)-1-(4-{[(3-chloro-4-méthoxyphényl)méthyl]amino}-5-{[(pyrimidin-2-yl)méthyl]carbamoyl}pyrimidin-2-yl)pyrrolidin-2-yl]méthyle, pour une utilisation dans le traitement selon la revendication 1, dans lequel la neuropathie optique ischémique antérieure est une neuropathie optique ischémique antérieure non artéritique.

3. 6-(nitrooxy)hexanoate de [(2S)-1-(4-{[(3-chloro-4-méthoxyphényl)méthyl]amino}-5-{[(pyrimidin-2-yl)méthyl]carbamoyl}pyrimidin-2-yl)pyrrolidin-2-yl]méthyle, pour une utilisation dans le traitement selon la revendication 1, dans lequel la neuropathie optique ischémique antérieure est une neuropathie optique ischémique antérieure artéritique.

4. 6-(nitrooxy)hexanoate de [(2S)-1-(4-{[(3-chloro-4-méthoxyphényl)méthyl]amino}-5-{[(pyrimidin-2-yl)méthyl]carbamoyl}pyrimidin-2-yl)pyrrolidin-2-yl]méthyle pour une utilisation dans le traitement selon l'une quelconque des revendications 1 à 3, dans lequel le 6-(nitrooxy)hexanoate de [(2S)-1-(4-{[(3-chloro-4-méthoxyphényl)méthyl]amino}-5-{[(pyrimidin-2-yl)méthyl]carbamoyl}pyrimidin-2-yl)pyrrolidin-2-yl]méthyle est administré sous forme de compositions pharmaceutiques ophtalmiques.

5. 6-(nitrooxy)hexanoate de [(2S)-1-(4-{[(3-chloro-4-méthoxyphényl)méthyl]amino}-5-{[(pyrimidin-2-yl)méthyl]carbamoyl}pyrimidin-2-yl)pyrrolidin-2-yl]méthyle pour une utilisation dans le traitement selon l'une quelconque des revendications 1 à 3, dans lequel le patient reçoit un traitement concomitant par corticostéroïdes.

6. Composition ophtalmique comprenant du 6-(nitrooxy)hexanoate de [(2S)-1-(4-{[(3-chloro-4-méthoxyphényl)méthyl]amino}-5-{[(pyrimidin-2-yl)méthyl]carbamoyl}pyrimidin-2-yl)pyrrolidin-2-yl]méthyle et un véhicule et/ou un support pharmaceutiquement acceptable pour une utilisation dans le traitement de la neuropathie optique ischémique antérieure chez un patient en ayant besoin, dans laquelle ladite composition est administrée à l'œil par voie topique ou par injection sous-conjonctivale.
